(19) Europäisches Patentamt
European Patent Office
Office européen des brevets

(11) EP 1 674 071 B1

(12) # FASCICULE DE BREVET EUROPEEN

(45) Date de publication et mention de la délivrance du brevet:
**28.07.2010 Bulletin 2010/30**

(21) Numéro de dépôt: **05292766.2**

(22) Date de dépôt: **21.12.2005**

(51) Int Cl.:
***A61K 8/41*** (2006.01) ***A61K 8/34*** (2006.01)
***A61K 8/42*** (2006.01) ***A61K 8/81*** (2006.01)
***A61K 8/90*** (2006.01) ***A61K 8/898*** (2006.01)
***A61Q 5/12*** (2006.01)

(54) **Composition comprenant un copolymère séquencé, une silicone aminée et un épaississant non polymérique et utilisations**

Zusammensetzung enthaltend ein Blockcopolymer, ein Aminosilikon und ein nicht-polymeres Verdickungsmittel und Verwendungen

Composition comprising a block copolymer, an aminosilicone and an non polymeric thickener and uses

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.12.2004 FR 0413800**

(43) Date de publication de la demande:
**28.06.2006 Bulletin 2006/26**

(73) Titulaire: **L'Oréal**
**75008 Paris (FR)**

(72) Inventeurs:
- **Panangatte, Lydia**
**78300 Poissy (FR)**
- **Giroud, Franck**
**92110 Clichy (FR)**
- **Dubief, Claude**
**78150 Le Chesnay (FR)**

(74) Mandataire: **Casalonga, Axel**
**Casalonga & Partners**
**Bayerstrasse 71/73**
**80335 München (DE)**

(56) Documents cités:

| | |
|---|---|
| **EP-A- 0 455 185** | **EP-A- 1 120 102** |
| **EP-A- 1 138 317** | **EP-A- 1 312 335** |
| **EP-A- 1 366 741** | **EP-A- 1 426 038** |
| **WO-A-2005/039517** | **FR-A- 2 548 019** |

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

EP 1 674 071 B1

## Description

La présente invention concerne des compositions non détergentes comprenant au moins un copolymère séquencé particulier comprenant au moins un premier bloc hydrophile et au moins un deuxième bloc qui est un bloc hydrophile différent du premier bloc hydrophile ou un bloc hydrophobe, le ou les blocs hydrophiles représentant au moins 30 % en poids du copolymère séquencé, au moins une silicone particulière et au moins un agent épaississant particulier.

Elle concerne aussi l'utilisation de ces compositions pour apporter du démêlage, du lissage et de la brillance aux fibres kératiniques.

Il est bien connu que des cheveux qui ont été sensibilisés, c'est-à-dire abîmés et/ou fragilisés, à des degrés divers sous l'action d'agents atmosphériques ou sous l'action de traitements mécaniques ou chimiques, tels que des colorations, des décolorations et/ou des permanentes, sont souvent difficiles à démêler et à coiffer et manquent de douceur.

La demande de brevet EP 0 455 185 décrit des microémulsions comprenant un composé aminé non soluble dans l'eau, un sel métallique ionisable et un ester oxyalkyléné.

La demande de brevet EP 1 426 038 décrit des compositions de teinture d'oxydation pour fibres kératiniques, comprenant un colorant d'oxydation, un polymère associatif et une silicone aminée.

La demande de brevet EP 1 138 317 décrit des compositions de coloration capillaires comprenant un noyau complexe capable de former un complexe avec un colorant acide.

La demande de brevet WO 2005/039517 décrit des compositions capillaires conditionnantes, comprenant un tensioactif cationique, un alcool gras, de l'argile modifiée par des groupements hydrophobes, un agent de traitement des cheveux et un copolymère séquencé à motifs d'oxyéthylène et d'oxypropylène.

La demande de brevet FR 2 548 019 décrit des compositions cosmétiques pour le traitement et les soins des cheveux, comprenant un agent de surface cationique dispersible dans l'eau, un polymère cationique quatemisé soluble dans l'eau du type ionène et un polymère cationique siliconé.

La demande de brevet EP 1 312 335 décrit des compositions cosmétiques capillaires comprenant une silicone aminée particulière et un agent conditionneur:

La demande de brevet EP 1 120 102 décrit des nanoémulsions comprenant des lipides amphiphiles et un polymère non ionique comportant au moins un séquence hydrophobe et au moins une séquence hydrophile.

Pour tenter de résoudre ce problème, l'homme de l'art utilise déjà, dans des compositions de soin des matières kératiniques telles que des cheveux, des agents conditionneurs, notamment des tensioactifs cationiques, des polymères cationiques ou des silicones.

Cependant, si ces compositions permettent, dans une certaine mesure, d' améliorer le démêlage et la douceur des cheveux, elles s'accompagnent malheureusement également, sur cheveux, de certains effets cosmétiques jugés indésirables, à savoir un alourdissement de la coiffure (manque de légèreté des cheveux), un manque de lissage (cheveux non homogènes de la racine à la pointe) et une insuffisance concernant la brillance.

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion inter-fibres affectant le coiffage. Ces inconvénients sont accentués dans le cas de cheveux fins, qui manquent de nervosité et de volume.

Les compositions cosmétiques actuelles contenant des agents conditionneurs ne donnent donc pas complètement satisfaction.

En outre, leur formulation repose sur l'utilisation des tensioactifs cationiques, peu tolérés par la faune et la flore aquatiques.

A l'issue de nombreux travaux, la demanderesse a découvert que les problèmes énoncés ci-dessus peuvent être résolus en appliquant sur cheveux une composition non détergente contenant dans un milieu cosmétiquement acceptable au moins un copolymère particulier à blocs hydrosoluble ou hydrodispersible autre qu'un copolymère siliconé, un polyuréthane ou un copolymère dont les blocs sont constitués uniquement d'oxyde d'éthylène et d'oxyde de propylène, au moins une silicone aminée et au moins un épaississant non polymérique choisi parmi les alcools gras comprenant une chaîne alkyle ou alkylène linéaire ou ramifiée en $C_{10}$-$C_{30}$et/ou les amides.

De manière surprenante et avantageuse, la demanderesse a mis en évidence que l'utilisation d'un groupe de copolymères séquencés particuliers, décrits plus en détail ci-après dans une composition non-détergente, en combinaison avec une silicone aminée et un épaississant non polymérique choisi parmi les alcools gras comprenant une chaîne alkyle ou alkylène linéaire ou ramifiée en $C_{10}$-$C_{30}$ et les amides permet de pallier les inconvénients des compositions de l'art antérieur.

Les compositions selon la présente demande permettent ainsi d'améliorer le démêlage des cheveux tout en conférant à la coiffure de bonnes propriétés cosmétiques telles que légèreté, lissage, brillance qui se maintiennent après

de multiples applications. Elles permettent également d'éviter les inconvénients généralement rencontrés lors de l'utilisation de polymères cationiques.

La présente invention a par conséquent pour objet une composition cosmétique non détergente comprenant, dans un milieu cosmétiquement acceptable,

- au moins un copolymère séquencé comprenant au moins un premier bloc hydrophile et au moins un deuxième bloc qui est un bloc hydrophile différent du premier bloc hydrophile ou un bloc hydrophobe, le ou les blocs hydrophiles représentant au moins 30 % en poids du copolymère séquencé, ce copolymère n'étant pas choisi parmi les copolymères séquencés d'oxyde d'éthylène et d'oxyde de propylène, les copolymères séquencés à motifs uréthane (polyuréthane) et les copolymères séquencés à motifs siloxane (copolymère siliconé) (voir revendication 1 pour la définition du polymère séquencé considéré).
- au moins une silicone aminée,
- au moins un agent épaississant non polymérique choisi parmi les alcools gras comprenant une chaîne alkyle ou alkylène linéaire ou ramifiée en $C_{10}$ - $C_{30}$ et les amides,
  ladite composition ne contenant pas plus de 4% en poids d'agents tensioactifs par rapport au poids total de la composition.

La présente demande concerne encore l'utilisation de la composition selon l'invention pour le démêlage, le lissage et la brillance des fibres kératiniques telles que les cheveux et le maintien de ces propriétés après des applications multiples.

D'autres caractéristiques, aspects, objets et avantages de la présente invention apparaîtront encore plus clairement à la lecture de la description et des exemples qui suivent.

Par « composition non détergente » au sens de la présente demande, on entend que la composition ne contient pas plus de 4% en poids d'agents tensio-actifs par rapport au poids total de la composition.

Dans le présent texte,

- les expressions « copolymère séquencé », « copolymère blocs » et « copolymères à blocs » seront utilisées de manière indifférente pour désigner le copolymère utilisé dans les compositions selon la présente demande,

- les expressions « silicone aminée » et « silicone aminofonctionnelle » seront utilisées de manière indifférente pour désigner la silicone aminée utilisée dans les compositions selon la présente demande.

Les copolymères séquencés utilisables selon la présente invention sont des copolymères diblocs, triblocs ou multiblocs, linéaires ou en étoile qui se solubilisent ou se dispersent dans l'eau spontanément ou par neutralisation.

Les copolymères séquencés utilisables selon la présente invention sont des copolymères hydrophiles ou hydrosolubles comprenant au moins deux blocs hydrophiles différents, ou des copolymères amphiphiles comportant à la fois des blocs hydrophobes et des blocs hydrophiles. Ces copolymères peuvent être anioniques, non ioniques ou amphotères, de préférence ils sont anioniques.

On entend par « monomère ou polymère hydrosoluble ou hydrodispersible » un monomère ou polymère qui, à la concentration de 0,1% en matière active dans l'eau à 25°C, conduit spontanément ou après neutralisation à l'aide d'un acide ou d'une base à une solution macroscopiquement homogène et transparente, c'est-à-dire ayant une valeur de transmittance à une longueur d'onde de 500 nm à travers un échantillon de 1 cm d'épaisseur d'au moins 70%, de préférence 80%.

Dans le cas contraire, le monomère ou le polymère est dit hydrophobe.

L'aptitude des copolymères séquencés amphiphiles à être dissous voire finement dispersés dans l'eau est liée à la proportion importante des séquences hydrophiles.

Cette proportion doit être d'au moins 30 % en poids, et elle est de préférence supérieure ou égale à 60 % en poids, sans pour autant dépasser 97 % en poids.

On entend par « bloc hydrophile » un bloc comprenant au moins 75 % en moles de monomères hydrosolubles et/ou hydrosolubilisables et/ou hydrodispersibles par neutralisation. Le bloc hydrophile peut donc comprendre jusqu'à 25% en moles d'un ou plusieurs monomères hydrophobes tels que définis ci-dessus. Cette proportion est de préférence inférieure ou égale à 10% en moles et de manière encore plus préférée inférieure ou égale à 5% en moles.

On entend par « bloc hydrophobe » un bloc comprenant au moins 75 % en moles de monomères hydrophobes. Le bloc hydrophobe peut donc comprendre jusqu'à 25% en moles d'un ou plusieurs monomères hydrosolubles ou hydrodispersibles tels que définis ci-dessus. Cette proportion est de préférence inférieure ou égale à 10% en moles et de manière encore plus préférée inférieure ou égale à 5% en moles.

Les copolymères séquencés utilisés englobent bien entendu également ceux dans lesquels les blocs hydrophiles et les blocs hydrophobes sont constitués exclusivement respectivement de monomères hydrosolubles ou hydrodispersibles et de monomères hydrophobes.

Ces blocs peuvent être des blocs homopolymères ou des blocs copolymères renfermant deux ou plus de deux monomères différents du même type.

La masse moléculaire moyenne en nombre de chaque bloc, qu'il soit hydrophile ou hydrophobe, copolymère ou homopolymère, est de préférence comprise entre 500 et 1000000, en particulier entre 500 et 500000. L'indice de polydispersité (Mw/Mn) du copolymère est compris entre 1,01 et 3,00 de préférence entre 1,10 et 2,50.

Les monomères hydrosolubles ou hydrodispersibles formant les blocs hydrophiles des copolymères séquencés utilisés dans la présente invention peuvent être de nature anionique, non-ionique ou cationique et peuvent être utilisés seuls ou sous forme de mélange contenant deux ou plusieurs monomères différents.

Les monomères hydrosolubles ou hydrodispersibles anioniques sont choisis parmi les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.

Les monomères hydrosolubles ou hydrodispersibles non-ioniques englobent l'acrylamide, les acrylamides N-alkylés en $C_{1-6}$ ou N,N-dialkylés en $C_{1-3}$, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Les monomères hydrosolubles ou hydrodispersibles cationiques englobent le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule

$$H_2C=CR_1\text{-}CO\text{-}X_2$$

dans laquelle

$R_1$ représente un atome d'hydrogène ou un groupe méthyle,

$X_2$ représente un groupe hydrocarboné linéaire ou ramifié en $C_{1-6}$ portant au moins une fonction amine primaire, secondaire, tertiaire ou au moins un atome d'azote quaternaire, ou un groupe de formule $NHR_2$ ou de formule $NR_2R_3$ où $R_2$ et $R_3$ représentent indépendamment l'un de l'autre chacun un groupe hydrocarboné en $C_{1-6}$, linéaire ou ramifié, portant au moins une fonction amine primaire, secondaire, tertiaire ou au moins un atome d'azote quaternaire.

Les monomères hydrophobes formant les blocs hydrophobes des copolymères séquencés sont choisis parmi les monomères vinylaromatiques tels que le styrène et ses dérivés alkylés comme le 4-butylstyrène, l'alpha-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en $C_{1-10}$, d'aryle en $C_{6-10}$ ou d'aralkyle en $C_{1-10}$ de préférence en $C_{6-10}$ et les méthacrylates d'alkyle en $C_{1-10}$, d'aryle en $C_{6-10}$ ou d'aralkyle en $C_{1-10}$ de préférence en $C_{6-10}$, comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule $CH_2=CH\text{-}O\text{-}R$ et les allyléthers de formule $CH_2=CH\text{-}CH_2\text{-}O\text{-}R$ où R représente un groupe alkyle en $C_{1-6}$, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les alpha-fluoroacrylates d'alkyle.

Les copolymères linéaires séquencés utilisés dans la présente invention peuvent être

- des copolymères diblocs de formule AB,
- des copolymères triblocs de formule ABA ou BAB et
- des copolymères multiblocs comprenant, au moins deux blocs hydrophiles et au moins deux blocs hydrophobes disposés en alternance, chaque A représentant un bloc hydrophile et chaque B représentant un bloc hydrophobe, les blocs A d'un même polymère pouvant être identiques ou différents et les blocs B d'un même polymère pouvant être identiques ou différents.

On préfère en particulier des copolymères diblocs et des copolymères triblocs comportant un bloc central hydrophile et deux blocs latéraux hydrophobes.

Les polymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes (*Atom Transfert Radical Polymerization* ou ATRP) (voir JACS, 117, page 5614 (1995), de Matyjasezwski *et al.*), la méthode des radicaux tels

que les nitroxydes (Georges et al., Macromolecules, 1993, 26, 2987) ou encore la voie par transfert de chaîne réversible avec addition-fragmentation (Radical Addition-Fragmentation chain Transfert) telle que le procédé MADIX (Macromolecular Design via the Interchange of Xanthate) (Charmot D., Corpart P., Adam H., Zard S.Z., Biadatti T., Bouhadir G., Macromol. Symp., 2000, 150, 23).

On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

Les polymères en étoile ou dendrimères sont des polymères et oligomères hautement ramifiés de structure chimique bien définie, on dit que ce sont des polymères hyperbranchés "parfaits". En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la N-ième génération sont les groupes fonctionnels terminaux des fuseaux de la N-ième génération ou génération terminale. De tels polymères sont décrits en particulier dans D.A. Tomalia, A.M. Naylor et W.A. Goddard III, Angewandte Chemie, Int. Ed. Eng1.29, 138-175 (1990) ; C.J. Hawker et J.M.J. Frechet, J.Am. Chem Soc., 112, 7638 (1990) ; B I Volt, Acta Polymer., 46, 87-99 (1995) ; N.Ardoin et D. Astruc. Bull. Soc. Chim. Fr 132, 875-909 (1995) ; G.R. Newkome, C.N. Moorefield, F. VÖgtle, Dendritic Molecules, VCH Verlagegesellschaft, 1996.

La quantité de copolymères séquencés dans les compositions de la présente invention dépend d'un grand nombre de paramètres parmi lesquels on peut citer la masse moléculaire des copolymères séquencés, le nombre et la taille des blocs hydrophiles et éventuellement hydrophobes, et éventuellement la viscosité de la composition que l'on souhaite obtenir.

A titre d'exemples, on utilise les copolymères suivants : poly(styrene-b-acide acrylique) ; poly(methyl-acrylamide-b-acide acrylique) ; copolymère dibloc poly(triméthylammonium ethyl acrylate quaternisé-b-acrylamide) ; copolymère tribloc poly(styrène-b-acide acrylique-b-styrène).

Les copolymères séquencés sont présents dans les compositions selon l'invention en une quantité allant de 0,01 à 20 % en poids de matière active, de préférence de 0,1 à 5 % en poids de matière active par rapport au poids de la composition.

Les compositions de la présente invention contiennent au moins une silicone aminée. On désigne par « silicone aminée » toute silicone comportant au moins une amine primaire, secondaire, tertiaire ou un groupement ammonium quaternaire. On peut ainsi citer :

a) les polysiloxanes répondant à la formule (1):

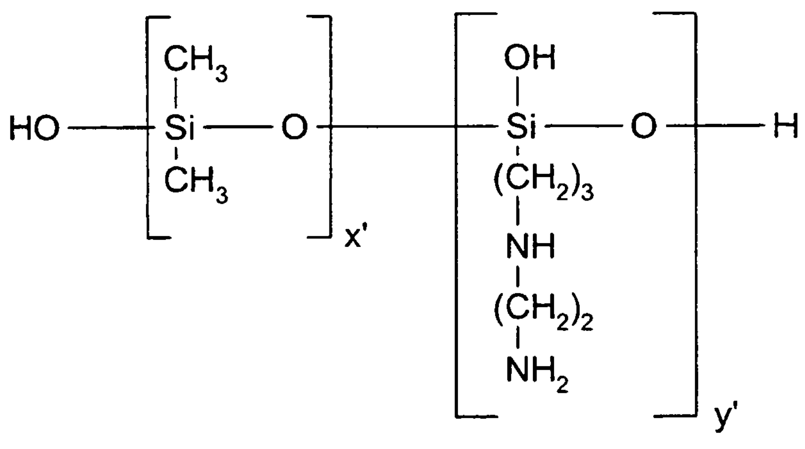

(I)

dans laquelle x' et y' sont des nombres entiers dépendant du poids moléculaire, généralement tels que ledit poids moléculaire moyen en poids est compris entre 5 000 et 500 000 environ ;

b) les silicones aminées répondant à la formule :

$$R'_aG_{3-a}\text{-}Si(OSiG_2)_n\text{-}(OSiG_bR'_{2-b})_m\text{-}O\text{-}SiG_{3-a}\text{-}R'_a \qquad (II)$$

dans laquelle :

G, identiques ou différents, désignent un atome d'hydrogène, ou un groupement phényle, OH, alkyle en $C_1$-$C_8$, par exemple méthyle, ou alcoxy en $C_1$-$C_8$, par exemple méthoxy
a identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R', identiques ou différents, désignent un radical monovalent de formule -CqH2qL dans laquelle q est un nombre de 2 à 8 et L est un groupement aminé éventuellement quaternisé choisi parmi les groupements :

- $NR''-Q-N(R'')_2$
- $N(R'')_2$
- $N^+(R'')_3$ A-
- $N^+H(R'')_2$ A-
- $N^+H_2(R'')$ A-
- $N(R'')-Q-N^+R''H_2$ A-
- $NR''-Q-N^+ (R'')_2H$ A-
- $NR''-Q-N^+ (R'')_3$ A-,

dans lesquels R'' peut désigner hydrogène, phényle, benzyle, ou un radical hydrocarboné saturé monovalent, par exemple un radical alkyle ayant de 1 à 20 atomes de carbone ; Q désigne un groupement de formule $C_rH_{2r}$, linéaire ou ramifié, r étant un entier allant de 2 à 6, de préférence de 2 à 4 ; et A- représente un ion halogénure tel que par exemple fluorure, chlorure, bromure ou iodure.

Un groupe de silicones aminées correspondant à cette définition est représentée par les silicones dénommées "triméthylsilylamodiméthicone", répondant à la formule :

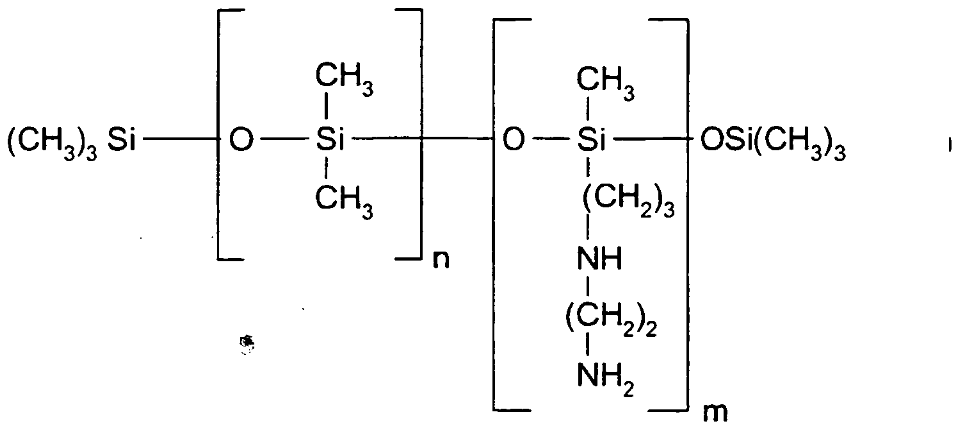

(III)

dans laquelle n et m ont les significations données ci-dessus (cf formule II).

De tels polymères sont décrits par exemple dans la demande de brevet EP-A-95238.

Un autre groupe de silicones aminées correspondant à cette définition est représentée par les silicones de formules (IV) ou (V) suivantes :

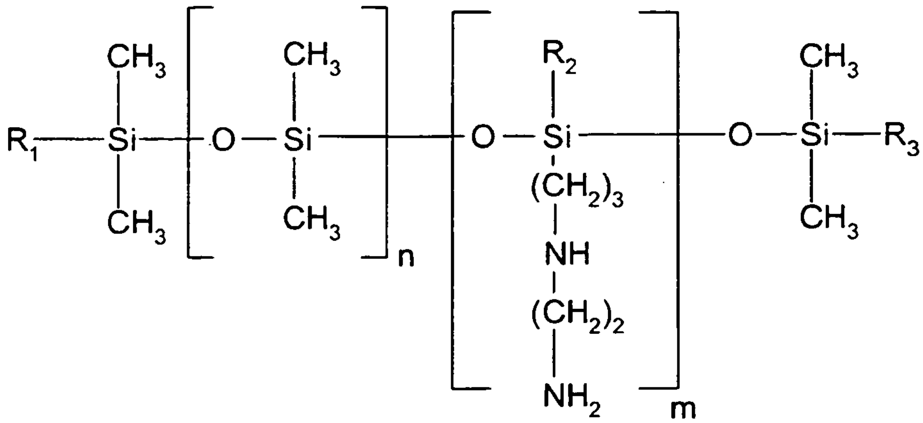

(IV)

dans laquelle :

m et n sont des nombres tels que la somme (n + m) peut varier notamment de 1 à 1 000 et en particulier de 50 à 250 et plus particulièrement de 100 à 200,

n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5,

$R_1$, $R_2$, $R_3$, identiques ou différents, représentent un radical hydroxy ou alcoxy en $C_1$-$C_4$,

l'un au moins des radicaux $R_1$ à $R_3$ désignant un radical alcoxy.

De préférence le radical alcoxy est un radical méthoxy.

Le rapport molaire Hydroxy/Alcoxy va de préférence de 0,2:1 à 0,4:1 et de préférence de 0,25:1 à 0,35:1 et plus particulièrement est égal à 0,3:1.

La masse moléculaire moyenne en poids de la silicone va de préférence de 2000 à 1000000 et encore plus particulièrement de 3500 à 200000.

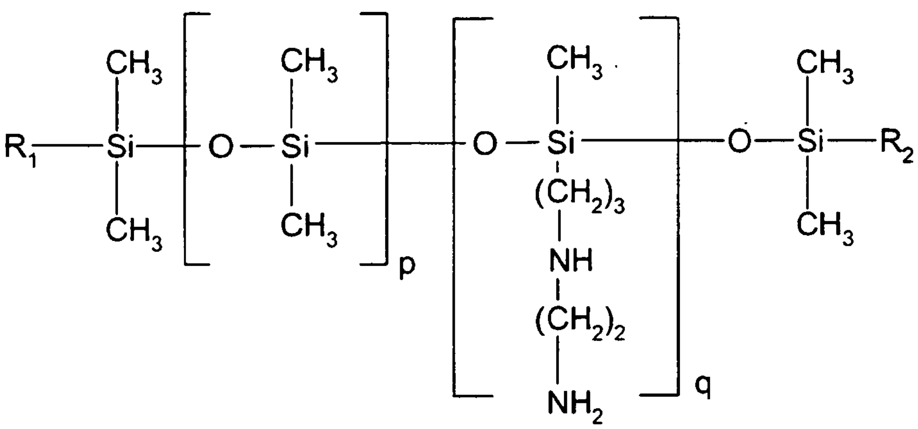

(V)

dans laquelle :

p et q sont des nombres tels que la somme (p + q) peut varier notamment de 1 à 1 000 et en particulier de 50 à 350, et plus particulièrement de 150 à 250,

p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$, $R_2$, différents, représentent un radical hydroxy ou alcoxy en $C_1$-$C_4$, l'un au moins des radicaux $R_1$ ou $R_2$ désignant un radical alcoxy.

De préférence le radical alcoxy est un radical méthoxy.

Le rapport molaire hydroxy/Alcoxy va généralement de 1:0,8 à 1:1,1 et de préférence de 1:0,9 à 1:1 et plus particulièrement est égal à 1:0,95.

La masse moléculaire moyenne en poids de la silicone va de préférence de 2000 à 200000 et encore plus particulièrement de 5000 à 100000 et plus particulièrement de 10000 à 50000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie

Les produits commerciaux correspondant à ces silicones de structure (IV) ou (V) peuvent inclure dans leur composition une ou plusieurs autres silicones aminées dont la structure est différente des formules (IV) ou (V).

Un produit contenant des silicones aminées de structure (IV) est proposé par la société WACKER sous la dénomination BELSIL® ADM 652.

Un produit contenant des silicones aminées de structure (V) est proposé par WACKER sous la dénomination Fluid WR 1300® .

Lorsque ces silicones aminées sont mises en oeuvre, une forme de réalisation particulièrement intéressante est leur utilisation sous forme d'émulsion huile dans eau.

L'émulsion huile dans eau peut comprendre un ou plusieurs tensioactifs.

Les tensioactifs peuvent être de toute nature mais de préférence cationique et/ou non ionique.

La taille moyenne en nombre des particules de silicone dans l'émulsion est généralement comprise entre 3 nm et 500 nanomètres .

De préférence, notamment comme silicones aminées de formule (V), on utilise des microémulsions dont la taille moyenne des particule est comprise entre 5 nm et 60 nanomètres (bornes incluses) et plus particulièrement entre 10 nm et 50 nanomètres (bornes incluses).

Ainsi, on peut utiliser selon l'invention les microémulsions de silicone aminée de formule (V) proposées sous les dénominations FINISH CT 96 E® ou SLM 28020® par la société WACKER.

De préférence la silicone aminée est choisie de façon telle que l'angle de contact avec l'eau d'un cheveu traité avec une composition contenant 2% (matière active) de ladite silicone selon l'invention soit compris entre 90° et 180° (bornes incluses) et de préférence compris entre 90 et 130° (bornes incluses).

Pour mesurer l'angle de contact, la silicone aminée est de préférence solubilisée ou dispersée dans un solvant de la silicone aminée ou de l'émulsion de silicone aminée (notamment l'hexaméthyldisiloxane ou l'eau selon l'hydrophilie de la silicone).

De préférence la composition contenant la ou les silicones aminées de formule (IV) ou (V) est telle que l'angle de contact avec l'eau d'un cheveu traité avec ladite composition est compris entre 90 et 180°(bornes incluses) et de préférence entre 90 et 130° (bornes incluses).

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90° (bornes incluses), hydrophobe lorsque cet angle est compris entre 90° et 180° (bornes incluses).

Le test s'effectue avec des mèches de cheveux naturels ayant été décolorés dans les mêmes conditions puis lavés.

Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique. La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, Γlv la tension interfaciale liquide / vapeur de l'eau en $J/m^2$ et θ l'angle de contact.

Le produit SLM 28020 de WACKER à 12% dans l'eau (soit 2% en silicone aminée) conduit à un angle de contact de 93° selon le test indiqué ci-dessus.

Le produit BELSIL ADM 652 de WACKER à 2% dans l'hexaméthyldisiloxane (soit 2% en silicone aminée) conduit à un angle de contact de 111° selon le test indiqué ci-dessus.

Un autre groupe de silicones aminées correspondant à cette définition est représenté par la formule suivante (VI):

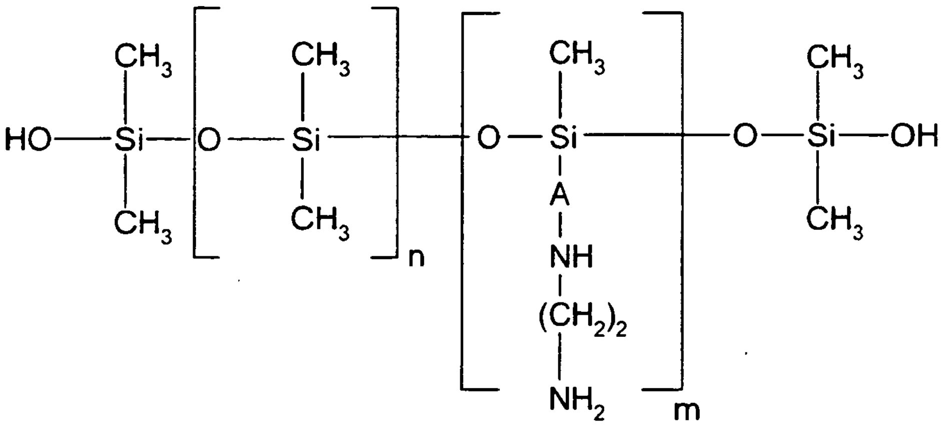

(VI)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

A désigne un radical alkylène linéaire ou ramifié ayant de 4 à 8 atomes de carbone et de préférence 4 atomes de carbone. Ce radical est de préférence linéaire.

La masse moléculaire moyenne en poids des silicones aminées va de préférence de 2000 à 1000000 et encore plus particulièrement de 3500 à 200000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Selon l'invention la viscosité à 25°C de la silicone aminée est notamment supérieure à 25000 cSt ($mm^2/s$) et de préférence va de 30000 à 200000 cSt ($mm^2/s$). et plus particulièrement de 30000 à 150000 cSt ($mm^2/s$).

Ces silicones aminées, ont de préférence un indice d'aminé inférieur ou égal à 0,4 meq./g, de préférence allant de 0,001 à 0,2 meq./g, et plus particulièrement allant de 0,01 à 0,1 meq./g.

L'indice d'amine est le nombre de milli-équivalents amine par gramme de composé. Cet indice est déterminé de façon tout à fait classique par des méthodes de titrage par indicateur coloré ou par titrage potentiométrique.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationiques et/ou non ioniques.

La taille moyenne en nombre des particules de silicone dans l'émulsion est généralement comprise entre 3 nm et 500 nanomètres, de préférence 5 nm et 300 nanomètres et plus particulièrement entre 10 nm et 275 nanomètres et encore plus préférentiellement entre 150 et 275 nanomètres.

Une silicone répondant à cette formule est par exemple la DC2-8299 de DOW CORNING.

c) les silicones aminées répondant à la formule (VII) :

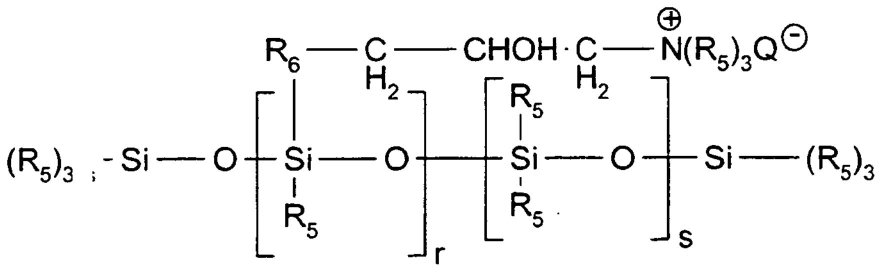

(VII)

dans laquelle :

$R_5$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;
$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC;
Q- est un anion tel qu'un ion halogénure, notamment chlorure ou un sel d'acide organique (acétate ...);
r représente une valeur statistique moyenne de 2 à 20 et en particulier de 2 à 8;
s représente une valeur statistique moyenne de 20 à 200 et en particulier de 20 à 50.

De telles silicones aminées sont décrites plus particulièrement dans le brevet US 4 185 087.

d) les silicones ammonium quaternaire de formule :

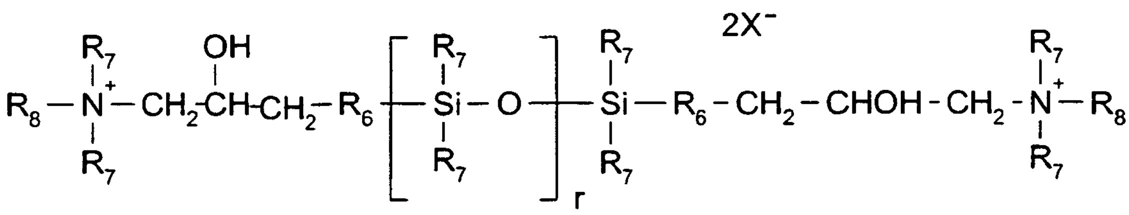

(VIII)

dans laquelle :

$R_7$, identiques ou différents, représentent un radical hydrocarboné monovalent ayant de 1 à 18 atomes de carbone, et en particulier un radical alkyle en $C_1$-$C_{18}$, un radical alcényle en $C_2$-$C_{18}$ ou un cycle comprenant 5 ou 6 atomes de carbone, par exemple méthyle ;
$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC;
$R_8$, identiques ou différents, représentent un atome d'hydrogène, un radical hydrocarboné monovalent ayant de 1 à 18 atomes de carbone, et en particulier un radical alkyle en $C_1$-$C_{18}$, un radical alcényle en $C_2$-$C_{18}$, un radical -$R_6$-NHCO$R_7$ ;
X- est un anion tel qu'un ion halogénure, notamment chlorure ou un sel d'acide organique (acétate ...);
r représente une valeur statistique moyenne de 2 à 200 et en particulier de 5 à 100 ;
Ces silicones sont par exemple décrites dans la demande EP-A-0530974.

e) les silicones aminées de formule :

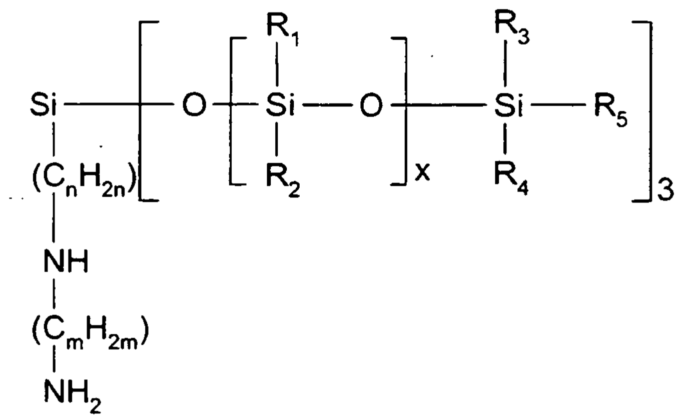

(IX)

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine soit compris entre 0,01 et 1 meq/g.
f) les silicones aminées polyoxyalkylénées de type $(XY)_i$ , X étant un bloc polysiloxane et Y étant un bloc polyoxyalkyléné comportant au moins un groupement amine pouvant, de préférence, être constituées d'unités répétitives de formule générale suivante:

$[SiMe_2\text{-}O\text{-} (SiMe_2O)_xSiMe_2 \text{ - } R\text{-}N(H)\text{-}R'\text{-}O \text{ -}(C_2H_4O)_a \text{ - } (C_3H_6O)_b \text{ - } R'\text{-}N(H)\text{-}R\text{-}]$ (X) dans laquelle :

- a est un nombre entier supérieur ou égal à 1, de préférence compris entre 5 et 200 et encore plus particulièrement entre 5 et 100 ;
- b est un nombre entier compris entre 0 et 200, de préférence compris entre 4 et 200 et encore plus particulièrement entre 5 et 100 ;
- x est un nombre entier compris entre 1 et 10000 et encore plus particulièrement entre 10 et 5000 ;
- R, identiques ou différents, représentent un groupe organique divalent qui est lié à l'atome de silicium adjacent par une liaison carbone-silicium et à un atome d'azote,
- R', identiques ou différents, représentent un groupe organique divalent qui est lié à l'atome d'oxygène adjacent par une liaison carbone-oxygène et à un atome d'azote,
  R est de préférence un radical hydrocarboné en $C_2$-$C_{12}$ comportant éventuellement un ou plusieurs hétéroatomes tels que l'oxygène. Plus particulièrement, R désigne un radical éthylène, propylène linéaire ou ramifié, butylène linéaire ou ramifié ou $-CH_2CH_2CH_2OCH(OH)CH_2-$.
  R' est de préférence un radical hydrocarboné en $C_2$-$C_{12}$ comportant éventuellement un ou plusieurs hétéroatomes tels que l'oxygène. Plus particulièrement R' désigne un radical alkylène divalent comme par exemple l'éthylène, le propylène linéaire ou ramifié

ou le butylène linéaire ou ramifié.

Les blocs siloxane représentent généralement entre 50 et 95% en moles par rapport au poids total de la silicone et plus particulièrement entre 70 et 85% en moles.
Le taux d'amine est généralement compris entre 0,02 et 0,5 meq/g de copolymère dans une solution à 30% dans le dipropylèneglycol et plus particulièrement entre 0,05 et 0,2.
Le poids moléculaire moyen en poids de la silicone de formule (X) est de préférence compris entre 5000 et 1000000 et encore plus particulièrement entre 10000 et 200000.
La silicone particulièrement visée par cette formule (X) est celle commercialisée sous la marque Silsoft A-843 Organosilicone Copolymer par OSI.
Les silicones particulièrement préférées conformément à l'invention sont les polysiloxanes à groupements aminés des familles de formules (I) à (VI).

La ou les silicones aminées sont utilisées de préférence en une quantité allant de 0,01 à 20% en poids par rapport au poids total de la composition. Plus préférentiellement, cette quantité va de 0,1 à 15% en poids et encore plus particulièrement de 0,5 à 10 % en poids par rapport au poids total de la composition.

Le ou les agents épaississants non polymériques sont choisis parmi les alcools gras et les amides.

Par « alcool gras » on entend, au sens de la présente demande, un alcool comprenant une chaîne alkyle ou alkylène linéaire ou ramifiée en $C_{10}$-$C_{30}$, de préférence les alcools gras utilisables de manière préférée sont les alcools stéarylique, myristique, cétylique, et leurs mélanges. Les mélanges d'alcool stéarylique et d'alcool cétylique sont utilisés de manière encore plus préférée.

Les amides utilisables de manière préférée dans les compositions selon la présente demande sont les amides à chaîne grasse en $C_{12}$-$C_{22}$, de préférence en $C_{16}$-$C_{18}$.Ces amides peuvent également être des alcanols amides.

Ce ou ces agents épaississants non polymériques sont utilisés en une quantité allant de 0,5 à 10%, de préférence 2 à 6% en poids par rapport au poids total de la composition.

Le milieu cosmétiquement acceptable peut être constitué uniquement d'eau ou d'un mélange d'eau et d'un ou de plusieurs solvants cosmétiquement acceptables miscibles avec l'eau tels que les alcools inférieurs en $C_1$-$C_4$, en particulier l'éthanol, l'isopropanol, le tertio-butanol et le n-butanol, et les polyols tels que le propylèneglycol ou le glycérol.

Le pH des compositions selon l'invention est généralement compris entre 3 et 9, de préférence 4 et 7.

Les compositions selon l'invention peuvent également contenir des additifs cosmétiques et/ou des adjuvants de formulation tels que les agents tensioactifs anioniques, cationiques, amphotères ou non-ioniques, les polymères anioniques, cationiques, amphotères ou non-ioniques supplémentaires autres que les copolymères séquencés utilisés en tant qu'agent essentiel de la composition, les agents nacrants, les agents opacifiants, les pigments et colorants, les parfums, les huiles végétales, animales ou synthétiques en particulier les silicones et organo-silicones supplémentaires autres que les silicones aminées utilisées en tant qu'agent essentiel de la composition, les gommes ou résines de silicone, les cires dont les céramides, les particules micro ou nanoparticules organiques ou minérales, les filtres UV organiques ou minéraux, les agents anti-radicalaires, les vitamines, les peptides, les protéines, les amino-acides, en particuliers les amino-acides basiques, les agents anti-pelliculaires, les agents antifongiques, les agents d'activation de la repousse du cheveu, les agents plastifiants, les agents d'ajustement et de fixation du pH, les agents anti-oxydants, les sels, les solvants, les agents conservateurs, les précurseurs de colorants capillaires et les agents oxydants.

Lorsqu'ils sont présents, la quantité de ces différents additifs cosmétiques et/ou des adjuvants de formulation va généralement de 0,001 à 20% en poids par rapport au poids total de la composition

L'homme de métier veillera à choisir les éventuels additifs et leur quantité de manière à ce qu'ils ne nuisent pas aux propriétés intéressantes des compositions de la présente invention.

Les compositions selon la présente demande pourront également comprendre un ester gras comme les esters de glycérol, par exemple les monostéarates ou monocétylate de glycérol, les esters d'acides et d'alcools gras comme les esters stéarylique, myristique, cétylique, palimitique des acides stéarylique, myristique, cétylique, palimitique.

Ces esters sont utilisés en une quantité allant de 0,1 à 5%, de préférence 0,5 à 3% en poids par rapport au poids total de la composition.

Les compositions de la présente invention peuvent se présenter sous n'importe quelle forme permettant une application aisée notamment sur les cheveux. Il s'agit de préférence de lotions épaissies, de gels aqueux ou hydro-alcooliques, de crèmes ou de pâtes plus ou moins dures.

Ces compositions peuvent être conditionnées dans un dispositif aérosol en présence d'un ou de plusieurs agents propulseurs. Ces agents propulseurs sont de préférence choisis parmi le diméthyléther, les alcanes en $C_{3-5}$, le 1,1-difluoroéthane, les mélanges de diméthyléther et d'alcanes en $C_{3-5}$ et les mélanges de 1,1-difluoroéthane et de diméthyléther et/ou d'alcanes en $C_{3-5}$.

Les exemples qui suivent illustrent la présente demande sans en limiter la portée.

## Exemples

I) Exemples comparatifs

Dans les compositions décrites ci-dessous, les pourcentages sont exprimés en poids de matière active.

Après shampooing

| CONSTITUANTS | Composition comparative 1 | Composition comparative 2 | Composition selon l'invention 3 | Composition selon l'invention 4 |
|---|---|---|---|---|
| Poly(styrene-b-acide acrylique)[1] | - | - | 1 % | - |
| Poly (methylacrylamide-b-acide acrylique) [2] | - | - | - | 1 % |
| Alcool cétéarylique | 3 % | 3% | 3 % | 3 % |
| ester cétylique | 1 % | 1 % | 1 % | 1 % |
| Amodimethicone DC939 (Dow Corning) | 1 % | 1 % | 1 % | 1 % |
| Chlorure de behenyltrimonium | - | 1 % | - | - |
| Acide citrique / soude | QS pH 6 | QS pH 6 | QS pH 6 | QS pH 6 |
| Conservateur | QS | QS | QS | QS |
| Parfum | QS | QS | QS | QS |
| Eau | QSP 100 % | QSP 100 % | QSP 100 % | QSP 100 % |

[1] Diblocs PS (2000g/mol)-PAA (300000g/mol) commercialisé par la société Polymer Source, Inc.
[2] Dibloc PAM (5000g/mol)-PAA (300000g/mol) commercialisé par la société Polymer Source, Inc.

12 g des compositions décrites ci-dessus sont appliqués sur différentes chevelures (cheveux caucasiens de 20 cm de long ayant subi au moins une coloration d'oxydation). Après deux minutes de pose, la chevelure est rincée à l'eau, puis un « brushing » au sèche-cheveux est réalisé. Le test est réalisé sur 10 modèles pour chaque composition. Les caractéristiques cosmétiques les plus pertinentes des chevelures sont évaluées par un panel de cinq experts.

L'application des après-shampooings 1, 2, 3 et 4 telle que décrite ci-dessus est renouvelée cinq fois. Avant chaque nouvelle application de l'après-shampooing, les chevelures sont lavées à l'aide d'un shampooing Elsève Multivitamines commercialisé par la société L'OREAL. Les critères cosmétiques sont évalués par le panel de cinq experts après les cinq applications répétées.

Les notes moyennes attribuées par le panel d'experts sont regroupées dans les tableaux suivants :

Tableau 1

| Composition n° | 1 (comparative) | 2 (comparative) | 3 (conforme) | 4 (conforme) |
|---|---|---|---|---|
| Démêlage sur cheveux humides[a] | 1.9 | 3.8 | 4.1 | 3.6 |
| Démêlage sur cheveux secs[b] | 1.6 | 4.0 | 3.9 | 3.7 |
| Lissage sur cheveux secs[c] | 1.6 | 3.3 | 3.8 | 3.4 |
| Brillance sur cheveux secs[d] | 2.4 | 3.5 | 3.9 | 3.5 |

L'application des après-shampooings telle que décrite ci-dessus est renouvelée dix fois. Avant chaque nouvelle application de l'après-shampooing, les chevelures sont lavées à l'aide d'un shampooing Elsève Multivitamines commercialisé par la société L'OREAL. Les critères cosmétiques sont à nouveau évalués par le panel de cinq experts après les dix applications répétées.

Les notes moyennes attribuées par le panel d'experts sont regroupées dans le tableau suivant :

Tableau 2 :

| Composition n° | 1 (comparative) | 2 (comparative) | 3 (conforme) | 4 (conforme) |
|---|---|---|---|---|
| Démêlage sur cheveux humides[a] | 2.2 | 3.9 | 4.0 | 3.5 |
| Démêlage sur cheveux secs[b] | 1.8 | 3.8 | 3.8 | 3.7 |
| Lissage sur cheveux secs[c] | 1.8 | 2.5 | 3.5 | 3.3 |
| Brillance sur cheveux secs[d] | 2.2 | 2.5 | 3.7 | 3.4 |
| [a] 0 : démêlage non réalisable<br>5 : démêlage en un seul passage de peigne<br>[b] 0 : démêlage non réalisable<br>5 : démêlage en un seul passage de peigne<br>[c] 0 : toucher inhomogène racine pointe<br>5 : toucher homogène racine pointe<br>[d] 0 : cheveux ternes<br>5 : cheveux brillants | | | | |

Les tableaux 1 et 2 ci-dessus montrent que la composition comparative 1 ne permet pas de conférer aux cheveux les propriétés cosmétiques attendues d'un produit de soin capillaire.

D'après le tableau 1, après applications, les compositions d'après-shampooings selon l'invention (compositions 3 et 4) donnent des propriétés cosmétiques comparables à la composition comparative n°2 comprenant un tensioactif cationique.

Après dix applications, le test (tableau 2) montre que les compositions de l'invention (compositions 3 et 4) apportent des propriétés cosmétiques supérieures à celles de l'état de la technique (composition 2). En particulier, on note pour les compositions de l'invention (compositions 3 et 4) un niveau de brillance et de lissage supérieurs à ceux obtenus avec l'état de la technique.

De façon surprenante, l'utilisation des l'après-shampooing selon l'invention comprenant un copolymère à blocs hydrosolubles ou hydrodispersibles permet d'apporter de bonnes propriétés cosmétiques et de les conserver à un très bon niveau lors de multi-applications.

II) Exemples de compositions

Les compositions conformes à l'invention suivantes ont également été préparées :

| Constituants | Composition 5 | Composition 6 |
|---|---|---|
| Alcool cétéarylique | 2,5% M.A. | 2,5% M.A. |
| Lauryl PEG/PPG-18/18 Methicone (Dow Corning 5200) | 0,23% M.A. | 0,23% M.A. |
| Ester cétylique | 0,5% M.A. | 0,5% M.A. |
| Copolymère (3) | 5% M.A. | - |
| Copolymère (4) | - | 1%M.A. |
| Amodimethicone (BELSIL ADM 6057 E -WACKER) | 1,4% M.A. | 1,4% M.A. |
| Acide citrique | - | Qs pH 6 |
| Eau | Qsp | Qsp |

Copolymère (3) : Copolymère Dibloc Poly(triméthylammonium ethyl acrylate quaternisé -b- acrylamide) 11k-30k commercialisé par la société Rhodia ;

Copolymère (4) : Copolymère Tribloc Poly(styrène-b-acide acrylique-b-styrène) 2K-12K-2K (P2971-SAAS commercialisé par la société Polymer Source, Inc.

## Revendications

**1.** Composition cosmétique, non détergente, comprenant, dans un milieu cosmétiquement acceptable :

- au moins un copolymère séquencé comprenant au moins un premier bloc hydrophile et au moins un deuxième bloc qui est un bloc hydrophile différent du premier bloc hydrophile ou un bloc hydrophobe, le ou les blocs hydrophiles représentant au moins 30 % en poids du copolymère séquencé à l'exclusion des copolymères séquencés d'oxyde d'éthylène et d'oxyde de propylène, des copolymères séquencés à motifs uréthanne et des copolymères séquencés à motifs siloxane,

  • et le ou les blocs hydrophiles étant formés de monomères hydrosolubles choisis parmi :

    - les monomères hydrosolubles ou hydrodispersibles anioniques choisis parmi les acides carboxyliques à insaturation éthylénique, l'acide 2-acrylamido-2-méthylpropanesulfonique, l'acide styrènesulfonique, l'acide vinylsulfonique et l'acide vinylphosphonique,
    - les monomères hydrosolubles ou hydrodispersibles non-ioniques choisis parmi l'acrylamide, les acrylamides N-alkylés en $C_{1-6}$ ou N,N-dialkylés en $C_{1-3}$, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle,
    - et les monomères hydrosolubles ou hydrodispersibles cationiques choisis parmi le chlorure de diméthyldiallylammonium, le chlorure de méthylvinylimidazolium, la 2-vinylpyridine, la 4-vinylpyridine, la 2-méthyl-5-vinylpyridine, la vinylamine, les monomères de formule

    $$H_2C{=}CR_1{-}CO{-}X_2$$

    dans laquelle
    $R_1$ représente un atome d'hydrogène ou un groupe méthyle,
    $X_2$ représente un groupe hydrocarboné linéaire ou ramifié en $C_{1-6}$ portant au moins une fonction amine primaire, secondaire, tertiaire ou au moins un atome d'azote quaternaire, ou un groupe de formule $NHR_2$ ou de formule $NR_2R_3$ où $R_2$ et $R_3$ représentent indépendamment l'un de l'autre chacun un groupe hydrocarboné en $C_{1-6}$, linéaire ou ramifié, portant au moins une fonction amine primaire, secondaire, tertiaire ou au moins un atome d'azote quaternaire,
    - ou un mélange de ces monomères hydrosolubles,

  • le ou les blocs hydrophobes étant formés de monomères insolubles dans l'eau choisis parmi les monomères vinylaromatiques, les diènes et les dérivés alkylés des diènes, le chloroprène, les acrylates d'alkyle en $C_{1-10}$, d'aryle en $C_{6-10}$ ou d'aralkyle en $C_{1-10}$, les méthacrylates d'alkyle en $C_{1-10}$, d'aryle en $C_{6-10}$ ou d'aralkyle en $C_{1-10}$, l'acétate de vinyle, les vinyléthers de formule $CH_2{=}CH{-}O{-}R$ et les allyléthers de formule $CH_2{=}CH{-}CH_2{-}O{-}R$ où R représente un groupe alkyle en $C_{1-6}$, l'acrylonitrile, le chlorure de vinyle, le chlorure de vinylidène, la caprolactone, l'éthylène, le propylène et les monomères vinyliques fluorés ou à chaîne perfluorée,

- au moins une silicone aminée,
- au moins un agent épaississant non polymérique choisi parmi les alcools gras comprenant une chaîne alkyle ou alkylène linéaire ou ramifiée en $C_{10}$-$C_{30}$ et les amides,

ladite composition ne contenant pas plus de 4 % en poids d'agents tensioactifs par rapport au poids total de la composition.

**2.** Composition selon la revendication 1 telle que les copolymères sont des copolymères diblocs, triblocs ou multiblocs, linéaires ou en étoile.

**3.** Composition selon l'une quelconque des revendications précédentes, **caractérisée par le fait que** le ou les blocs hydrophiles représentent au moins 60 % en poids du copolymère séquencé.

**4.** Composition selon l'une des revendications précédentes, **caractérisée par le fait que** le copolymère séquencé

est linéaire et choisi parmi les copolymères diblocs, les copolymères triblocs et les copolymères multiblocs.

5. Composition selon la revendication 4, **caractérisée par le fait que** le copolymère linéaire séquencé est choisi parmi les copolymères diblocs et les copolymères triblocs comportant un bloc central hydrophile et deux blocs latéraux hydrophobes.

6. Composition selon l'une quelconque des revendications précédentes, **caractérisée par le fait que** les blocs hydrophiles contiennent jusqu'à 25 % en moles, de préférence jusqu'à 10 % en moles, et idéalement jusqu'à 5 % en moles, d'un ou de plusieurs monomères insolubles dans l'eau selon la revendication 1.

7. Composition selon l'une quelconque des revendications précédentes, **caractérisée par le fait que** le ou les blocs hydrophobes contiennent jusqu'à 25 % en moles, de préférence jusqu'à 10 % en moles, et idéalement jusqu'à 5 % en moles, d'un ou de plusieurs monomères hydrosolubles ou hydrodispersibles selon la revendication 1.

8. Composition selon l'une quelconque des revendications précédentes, **caractérisée par le fait que** le ou les copolymères séquencés sont choisis parmi les poly(styrene-b-acide acrylique); poly(methyl-acrylamide-b-acide acrylique); copolymère dibloc poly(triméthylammonium ethyl acrylate quaternisé-b-acrylamide) ; copolymère tribloc poly (styrène-b-acide acrylique-b-styrène).

9. Composition selon l'une quelconque des revendications précédentes, **caractérisée par le fait que** le ou les copolymères séquencés sont présents à raison de 0,01 à 20 % en poids, de préférence à raison de 0,1 à 5 % en poids rapporté à la composition.

10. Composition selon l'une quelconque des revendications précédentes, **caractérisée par** la silicone aminée est choisie parmi

a) les polysiloxanes répondant à la formule :

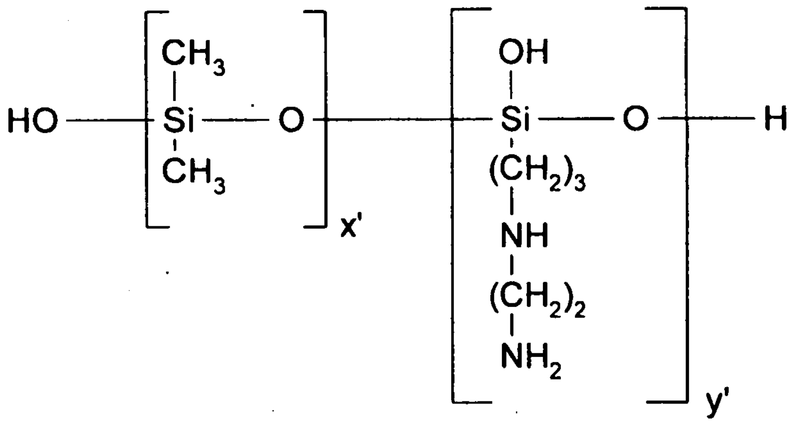

(I)

dans laquelle x' et y' sont des nombres entiers tels que ledit poids moléculaire moyen en poids est compris entre 5 000 et 500 000 environ ;
b) les silicones aminées répondant à la formule:

$$R'_aG_{3-a}\text{-}Si(OSiG_2)_n\text{-}(OSiG_bR'_{2-b})_m\text{-}O\text{-}SiG_{3-a}\text{-}R'_a \quad \text{(II)}$$

dans laquelle :

G, identiques ou différents, désignent un atome d'hydrogène, ou un groupement phényle, OH, ou alkyle en $C_1$-$C_8$, ou alcoxy en $C_1$-$C_8$,
a, identiques ou différents, désignent le nombre 0 ou un nombre entier de 1 à 3,
b, désigne 0 ou 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner

un nombre de 1 à 2 000, et notamment de 1 à 10;
R', identiques ou différents, désignent un radical monovalent de formule - CqH2qL dans laquelle q est un nombre de 2 à 8 et L est un groupement aminé éventuellement quaternisé choisi parmi les groupements :

$-NR''-Q-N(R'')_2$
$-N(R'')_2$
$-N^+(R'')_3$ A-
$-N^+H(R'')_2$ A-
$-N^+H_2(R'')$ A-
$-N(R'')-Q-N^+R''H_2$ A-
$-NR''-Q-N^+ (R'')_2H$ A-
$-NR''-Q-N^+ (R'')_3$ A-,

dans lesquels R" peut désigner hydrogène, phényle, benzyle, ou un radical hydrocarboné saturé monovalent, Q désigne un groupement de formule $C_rH_{2r}$, linéaire ou ramifié, r étant un entier allant de 2 à 6, de préférence de 2 à 4 ; et A-représente un ion halogénure

c) les silicones aminées répondant à la formule :

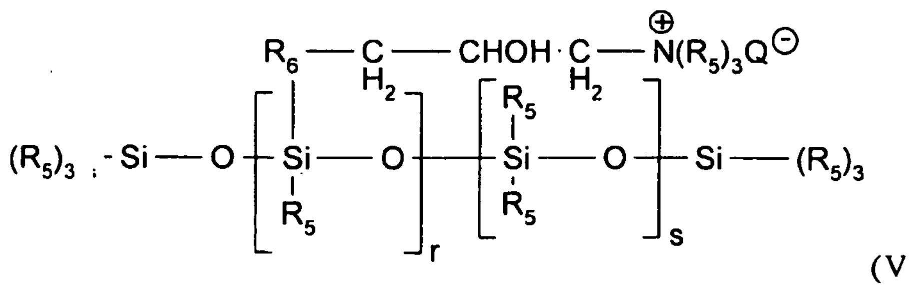

(VII)

dans laquelle :

$R_5$ représente un radical hydrocarboné monovalent ayant de 1 à 18 atomes de carbone,
$R_6$ représente un radical hydrocarboné divalent relié au Si par une liaison SiC;
$Q^-$ est un anion tel qu'un ion halogénure ou un sel d'acide organique ;
r représente une valeur statistique moyenne de 2 à 20 ;
s représente une valeur statistique moyenne de 20 à 200 ;

d) les silicones ammonium quaternaire de formule (VIII) :

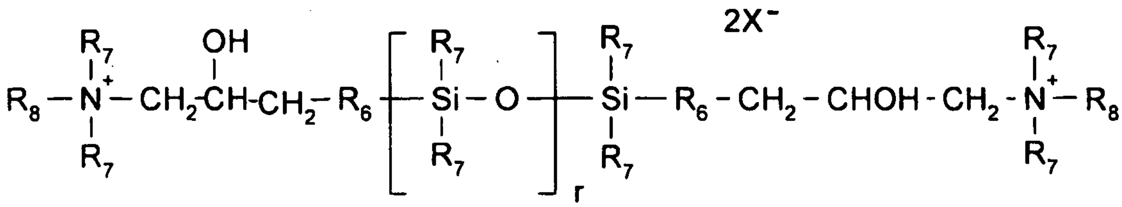

(VIII)

dans laquelle :

$R_7$, identiques ou différents, représentent un radical hydrocarboné monovalent ayant de 1 à 18 atomes de carbone ;
$R_6$ représente un radical hydrocarboné divalent relié au Si par une liaison SiC;
$R_8$, identiques ou différents, représentent un atome d'hydrogène, un radical hydrocarboné monovalent

ayant de 1 à 18 atomes de carbone;
X- est un anion tel qu'un ion halogénure ou un sel d'acide organique ;
r représente une valeur statistique moyenne de 2 à 200 ;

e) les silicones aminées de formule (IX) :

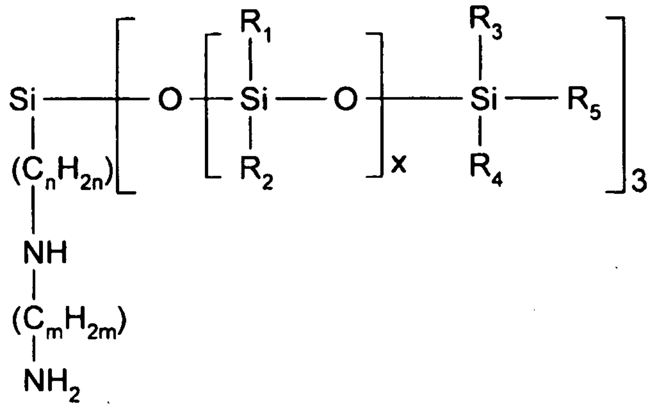

(IX)

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine soit compris entre 0,01 et 1 meq/g;
f) les silicones aminées polyoxyalkylénées de type (XY)i , X étant un bloc polysiloxane et Y étant un bloc polyoxyalkyléné comportant au moins un groupement amine pouvant, de préférence, être constituées d'unités répétitives de formule générale suivante:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (X)$$

dans laquelle :

- a est un nombre entier supérieur ou égal à 1, de préférence compris entre 5 et 200 et encore plus particulièrement entre 5 et 100.
- b est un nombre entier compris entre 0 et 200, de préférence compris entre 4 et 200 et encore plus particulièrement entre 5 et 100
- x est un nombre entier compris entre 1 et 10000 et encore plus particulièrement entre 10 et 5000 ;
- R, identiques ou différents, représentent un groupe organique divalent qui est lié à l'atome de silicium adjacent par une liaison carbone-silicium et à un atome d'azote.
- R', identiques ou différents, représentent un groupe organique divalent qui est lié à l'atome d'oxygène adjacent par une liaison carbone-oxygène et à un atome d'azote.

**11.** Composition selon l'une quelconque des revendications précédentes, **caractérisée par le fait que** la ou les silicones aminées sont présentes à raison de 0,01 à 20 % en poids, de préférence à raison de 0,1 à 15 % en poids

**12.** Composition selon l'une quelconque des revendications précédentes, **caractérisée par le fait que** les alcools gras sont choisis parmi les alcools stéarylique, myristique, cétylique, et leurs mélanges.

**13.** Composition selon l'une quelconque des revendications précédentes, **caractérisée par le fait que** les amides sont les amides à chaîne grasse en $C_{12}$-$C_{22}$, de préférence en $C_{16}$-$C_{18}$.

**14.** Composition selon l'une quelconque des revendications précédentes, **caractérisée par le fait que** le ou les agents épaississants non polymériques sont utilisés en une quantité allant de 0,5 à 10%, de préférence 2 à 6% en poids

par rapport au poids total de la composition

**15.** Composition selon l'une quelconque des revendications précédentes, **caractérisée par le fait qu'**elle contient en outre des additifs cosmétiques et/ou des adjuvants de formulation tels que les agents tensioactifs anioniques, cationiques, amphotères ou non-ioniques, les polymères anioniques, cationiques, amphotères ou non-ioniques supplémentaires, les agents nacrants, les agents opacifiants, les pigments et colorants, les parfums, les huiles végétales, animales ou synthétiques en particulier les silicones et organo-silicones supplémentaires, les gommes ou résines de silicone, les cires dont les céramides, les particules micro ou nanoparticules organiques ou minérales, les filtres UV organiques ou minéraux, les agents anti-radicalaires, les vitamines, les peptides, les protéines, les amino-acides, en particuliers les amino-acides basiques, les agents anti-pelliculaires, les agents antifongiques, les agents d'activation de la repousse du cheveu, les agents plastifiants, les agents d'ajustement et de fixation du pH, les agents anti-oxydants, les sels, les solvants, les agents conservateurs, les précurseurs de colorants capillaires et les agents oxydants.

**16.** Composition selon l'une quelconque des revendications précédentes, **caractérisée par le fait qu'**elle se présente sous forme de lotion épaissie, de gel, de crème ou de pâte.

**17.** Utilisation de la composition selon l'une des revendications précédentes pour le démêlage, le lissage et la brillance des fibres kératiniques telles que les cheveux.

**18.** Utilisation de la composition selon l'une des revendications précédentes pour conférer à la coiffure de bonnes propriétés cosmétiques qui se maintiennent après de multiples applications.

## Claims

**1.** Nondetergent cosmetic composition comprising, in a cosmetically acceptable medium:

- at least one block copolymer comprising at least a first hydrophilic block and at least a second block that is a hydrophilic block other than the first hydrophilic block, or a hydrophobic block, the hydrophilic block(s) representing at least 30% by weight of the block copolymer, with the exclusion of block copolymers of ethylene oxide and of propylene oxide, block copolymers comprising urethane units and block copolymers comprising siloxane units,

  • and the hydrophilic block(s) being formed from water-soluble monomers chosen from:

    - anionic water-soluble or water-dispersible monomers chosen from ethylenically unsaturated carboxylic acids, 2-acrylamido-2-methylpropanesulphonic acid, styrenesulphonic acid, vinylsulphonic acid and vinylphosphonic acid,
    - nonionic water-soluble or water-dispersible monomers chosen from acrylamide, $C_{1-6}$ N-alkyl or $C_{1-3}$ N,N-dialkyl acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate,
    - and cationic water-soluble or water-dispersible monomers chosen from dimethyldiallylammonium chloride, methylvinylimidazolium chloride, 2-vinylpyridine, 4-vinylpyridine, 2-methyl-5-vinylpyridine, vinylamine, and the monomers of formula

$$H_2C=CR_1-CO-X_2$$

in which
$R_1$ represents a hydrogen atom or a methyl group, $X_2$ represents a $C_{1-6}$ linear or branched hydrocarbon-based group bearing at least one primary, secondary or tertiary amine function or at least one quaternary nitrogen atom, or a group of formula $NHR_2$ or of formula $NR_2R_3$ where $R_2$ and $R_3$ each represent, independently of one another, a $C_{1-6}$ linear or branched hydrocarbon-based group bearing at least one primary, secondary or tertiary amine function or at least one quaternary nitrogen atom,
- or a mixture of these water-soluble monomers,

• the hydrophobic block(s) being formed from water-insoluble monomers chosen from vinylaromatic monomers, dienes and alkylated derivatives of dienes, chloroprene, $C_{1-10}$ alkyl, $C_{6-10}$ aryl or $C_{1-10}$ aralkyl acrylates, $C_{1-10}$ alkyl, $C_{6-10}$ aryl or $C_{1-10}$ aralkyl methacrylates, vinyl acetate, vinyl ethers of formula $CH_2$=CH-O-R and allyl ethers of formula $CH_2$=CH-$CH_2$-O-R where R represents a $C_{1-6}$ alkyl group, acrylonitrile, vinyl chloride, vinylidene chloride, caprolactone, ethylene, propylene, and vinyl monomers that are fluorinated or that contain a perfluoro chain,

- at least one amino silicone,
- at least one nonpolymeric thickener chosen from fatty alcohols comprising a linear or branched $C_{10}$-$C_{30}$ alkyl or alkylene chain, and amides,

said composition containing not more than 4% by weight of surfactants relative to the total weight of the composition.

**2.** Composition according to Claim 1, such that the copolymers are linear or star, diblock, triblock or multiblock copolymers.

**3.** Composition according to either one of the preceding claims, **characterized in that** the hydrophilic block(s) represent (s) at least 60% by weight of the block copolymer.

**4.** Composition according to one of the preceding claims, **characterized in that** the block copolymer is linear and is chosen from diblock copolymers, triblock copolymers and multiblock copolymers.

**5.** Composition according to Claim 4, **characterized in that** the linear block copolymer is chosen from diblock copolymers and triblock copolymers comprising a hydrophilic central block and two hydrophobic side blocks.

**6.** Composition according to any one of the preceding claims, **characterized in that** the hydrophilic blocks contain up to 25 mol%, preferably up to 10 mol%, and ideally up to 5 mol%, of one or more water-insoluble monomers according to Claim 1.

**7.** Composition according to any one of the preceding claims, **characterized in that** the hydrophobic block(s) contain (s) up to 25 mol%, preferably up to 10 mol%, and ideally up to 5 mol%, of one or more water-soluble or water-dispersible monomers according to Claim 1.

**8.** Composition according to any one of the preceding claims, **characterized in that** the block copolymer(s) is (are) chosen from poly(styrene-b-acrylic acid); poly(methylacrylamide-b-acrylic acid); poly(quaternized trimethylammonium ethyl acrylate-b-acrylamide) diblock copolymer; poly(styrene-b-acrylic acid-b-styrene) triblock copolymer.

**9.** Composition according to any one of the preceding claims, **characterized in that** the block copolymer(s) is (are) present in a proportion of 0.01 to 20% by weight, preferably in a proportion of 0.1 to 5% by weight, relative to the hair composition.

**10.** Composition according to any one of the preceding claims, **characterized in that** the amino silicone is chosen from

a) polysiloxanes corresponding to the formula:

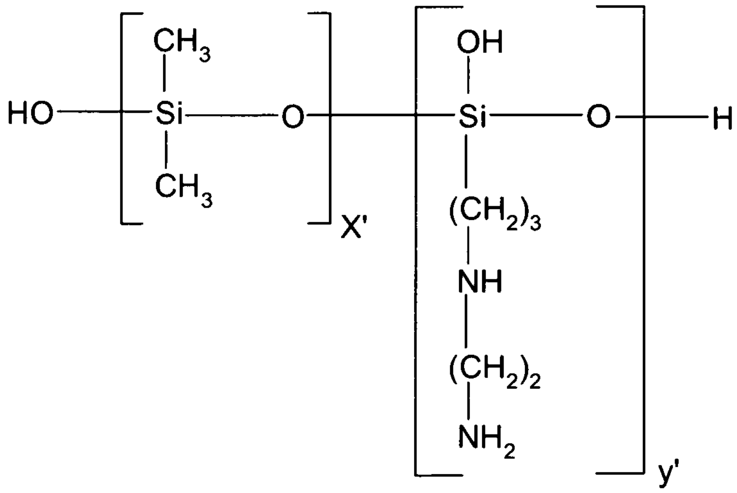

(I)

in which x' and y' are integers such that said weight-average molecular weight is between approximately 5000 and 500 000;

b) amino silicones corresponding to the formula:

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(II)}$$

in which:

G, which may be identical or different, denote a hydrogen atom, or a phenyl, OH, $C_1$-$C_8$ alkyl or $C_1$-$C_8$ alkoxy group,
a, which may be identical or different, denote the number 0 or an integer from 1 to 3,
b, denotes 0 or 1,
m and n are numbers such that the sum (n + m) can range especially from 1 to 2000, and in particular from 50 to 150, it being possible for n to denote a number from 0 to 1999, and especially from 49 to 149, and it being possible for m to denote a number from 1 to 2000, and especially from 1 to 10;
R', which may be identical or different, denote a monovalent radical of formula -CqH2qL in which q is a number from 2 to 8 and L is an optionally quaternized amino group chosen from the groups:

$-NR''-Q-N(R'')_2$
$-N(R'')_2$
$-N^+(R'')_3$ A-
$-N^+H(R'')_2$ A-
$-N^+H_2$ (R'') A-
$-N(R'')-Q-N^+R''H_2$ A-
$-NR''-Q-N^+ (R'')_2H$ A-
$-NR''-Q-N^+ (R'')_3$ A-,

in which R'' can denote hydrogen, phenyl, benzyl, or a monovalent saturated hydrocarbon-based radical, Q denotes a linear or branched group of formula $C_rH_{2r}$, r being an integer ranging from 2 to 6, preferably from 2 to 4; and A- represents a halide ion;

c) amino silicones corresponding to the formula:

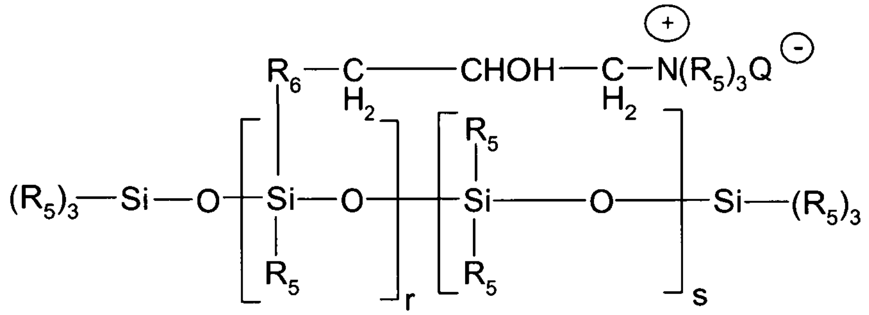

(VII)

in which:

$R_5$ represents a monovalent hydrocarbon-based radical having from 1 to 18 carbon atoms,
$R_6$ represents a divalent hydrocarbon-based radical connected to the Si via an SiC bond;
Q- is an anion such as a halide ion or an organic acid salt;
r represents a mean statistical value from 2 to 20;
s represents a mean statistical value from 20 to 200;

d) quaternary ammonium silicones of formula (VIII):

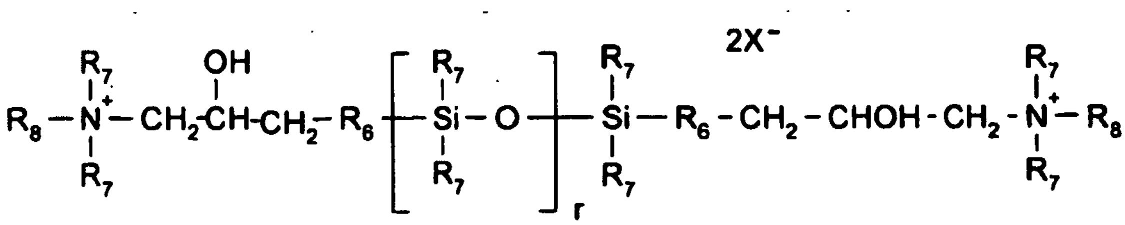

(VIII)

in which:

$R_7$, which may be identical or different, represent a monovalent hydrocarbon-based radical having from 1 to 18 carbon atoms;
$R_6$ represents a divalent hydrocarbon-based radical connected to the Si via an SiC bond;
$R_8$, which may be identical or different, represent a hydrogen atom, or a monovalent hydrocarbon-based radical having 1 to 18 carbon atoms;
X- is an anion such as a halide ion or an organic acid salt;
r represents a mean statistical value from 2 to 200;

e) amino silicones of formula (IX):

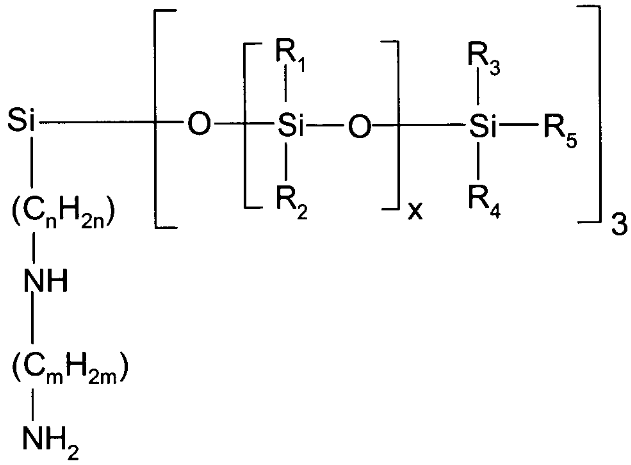

(IX)

in which:

- $R_1$, $R_2$, $R_3$ and $R_4$, which may be identical or different, denote a $C_1$-$C_4$ alkyl radical or a phenyl group,
- $R_5$ denotes a $C_1$-$C_4$ alkyl radical or a hydroxyl group,
- n is an integer ranging from 1 to 5,
- m is an integer ranging from 1 to 5,

and in which x is chosen such that the amine number is between 0.01 and 1 meq/g;

f) polyoxyalkylenated amino silicones of $(XY)_i$ type, X being a polysiloxane block and Y being a polyoxyalkylenated block comprising at least one amine group that may preferably consist of repeat units of the general formula below:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (X)$$

in which:

- a is an integer greater than or equal to 1, preferably between 5 and 200, and even more particularly between 5 and 100;
- b is an integer between 0 and 200, preferably between 4 and 200, and even more particularly between 5 and 100;
- x is an integer between 1 and 10 000, and even more particularly between 10 and 5000;
- R, which may be identical or different, represent a divalent organic group which is linked to the adjacent silicon atom via a carbon-silicon bond and to a nitrogen atom;
- R', which may be identical or different, represent a divalent organic group which is linked to the adjacent oxygen atom via a carbon-oxygen bond and to a nitrogen atom.

**11.** Composition according to any one of the preceding claims, **characterized in that** the amino silicone(s) is (are) present in a proportion of 0.01 to 20% by weight, preferably in a proportion of 0.1 to 15% by weight.

**12.** Composition according to any one of the preceding claims, **characterized in that** the fatty alcohols are chosen from stearyl alcohol, myristic alcohol and cetyl alcohol, and mixtures thereof.

**13.** Composition according to any one of the preceding claims, **characterized in that** the amides are amides comprising a $C_{12}$-$C_{22}$, preferably $C_{16}$-$C_{18}$, fatty chain.

**14.** Composition according to any one of the preceding claims, **characterized in that** the nonpolymeric thickener(s) is (are) used in an amount ranging from 0.5 to 10%, preferably 2 to 6% by weight, relative to the total weight of the composition.

**15.** Composition according to any one of the preceding claims, **characterized in that** it also contains cosmetic additives and/or formulation adjuvants such as anionic, cationic, amphoteric or nonionic surfactants, additional anionic, cationic, amphoteric or nonionic polymers, pearlescent agents, opacifiers, pigments and dyes, fragrances, plant, animal or synthetic oils, in particular additional silicones and organosilicones, silicone gums or resins, waxes, including ceramides, organic or mineral microparticles or nanoparticles, organic or mineral UV-screening agents, free-radical scavengers, vitamins, peptides, proteins, amino acids, in particular basic amino acids, antidandruff agents, antifungal agents, agents for activating hair regrowth, plasticizers, agents for adjusting and fixing the pH, antioxidants, salts, solvents, preserving agents, hair dye precursors and oxidizing agents.

**16.** Composition according to any one of the preceding claims, **characterized in that** it is in the form of a thickened lotion, of a gel, of a cream or of a paste.

**17.** Use of the composition according to one of the preceding claims, for the disentangling, the smoothing and the sheen of keratin fibres such as the hair.

**18.** Use of the composition according to one of the preceding claims, for giving the hairstyle good cosmetic properties that are maintained after multiple applications.

**Patentansprüche**

**1.** Nichtreinigende kosmetische Zusammensetzung, in einem kosmetisch akzeptablen Medium enthält:

- mindestens ein Sequenzcopolymer, das mindestens einen ersten hydrophilen Block und mindestens einen zweiten Block aufweist, bei dem es sich um einen hydrophilen Block, der von dem ersten hydrophilen Block verschieden ist, oder einen hydrophoben Block handelt, wobei der hydrophile Block oder die hydrophilen Blöcke mindestens 30 Gew.-% des Sequenzcopolymers ausmachen, wobei Sequenzcopolymere von Ethylenoxid und Propylenoxid, Sequenzcopolymere, die Urethaneinheiten enthalten, und Sequenzcopolymere, die Siloxaneinheiten enthalten, ausgenommen sind;

  • wobei der oder die hydrophilen Blöcke aus wasserlöslichen Monomeren gebildet sind, die ausgewählt sind unter:

    - wasserlöslichen oder in Wasser dispergierbaren, anionischen Monomeren, die unter den Carbonsäuren mit ethylenisch ungesättigter Bindung, 2-Acrylamido-2-methylpropansulfonsäure, Styrolsulfonsäure, Vinylsulfonsäure und Vinylphosphonsäure ausgewählt sind;
    - wasserlöslichen oder in Wasser dispergierbaren, nichtionischen Monomeren, die unter Acrylamid, N-Alkyl($C_{1-6}$)acrylamiden oder N,N-Dialkyl($C_{1-3}$)acrylamiden, Polyethylenglycolacrylat, Polyethylenglycolmethacrylat, N-Vinylacetamid, N-Methyl-N-vinylacetamid, N-Vinylformamid, N-Methyl-N-vinylformamid, N-Vinyllactamen, die eine cyclische Gruppe mit 4 bis 9 Kohlenstoffatomen aufweisen, Vinylalkohol, Ethylenoxid, Hydroxyethylacrylat, Hydroxypropylacrylat, Hydroxyethylmethacrylat und Hydroxypropylmethacrylat ausgewählt sind;
    - wasserlöslichen oder in Wasser dispergierbaren, kationischen Monomeren, die unter Dimethyldiallylammoniumchlorid, Methylvinylimidazoliumchlorid, 2-Vinylpyridin, 4-Vinylpyridin, 2-Methyl-5-vinylpyridin, Vinylamin, Monomeren der folgenden Formel ausgewählt sind:

$$H_2C=CR_1-CO-X_2$$

    worin bedeuten:

      $R_1$ ein Wasserstoffatom oder die Methylgruppe,
      $X_2$ eine geradkettige oder verzweigte Kohlenwasserstoffgruppe mit 1 bis 6 Kohlenstoffatomen, die mindestens eine primäre, sekundäre, tertiäre Aminofunktion oder mindestens ein quartäres Stickstoffatom aufweist, eine Gruppe der Formel $NHR_2$ oder der Formel $NR_2R_3$, wobei $R_2$ und $R_3$ unabhängig voneinander eine geradkettige oder verzweigte $C_{1-6}$-Kohlenwasserstoffgruppe bedeuten, die mindestens eine primäre, sekundäre, tertiäre Aminofunktion oder mindestens ein quartäres Stickstoffatom aufweist;

    - oder einem Gemisch dieser wasserlöslichen Monomere;

• wobei der oder die hydrophoben Blöcke aus in Wasser unlöslichen Monomeren gebildet sind, die unter den vinylaromatischen Monomeren, Dienen und alkylierten Dienderivaten, Chloropren, $C_{1-10}$-Alkylacrylaten, $C_{6-10}$-Arylacrylaten oder $C_{1-10}$-Aralkylacrylaten, $C_{1-10}$-Alkylmethacrylaten, $C_{6-10}$-Arylmethacrylaten oder $C_{1-10}$-Aralkylmethacrylaten, Vinylacetat, Vinylethern der Formel $CH_2$=CH-O-R und Allylethern der Formel $CH_2$=CH-$CH_2$-O-R, worin R eine $C_{1-6}$-Alkylgruppe bedeutet, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind;

- mindestens ein aminiertes Silicon,
- mindestens ein nicht polymeres Verdickungsmittel, das unter den Fettalkoholen, die eine lineare oder verzweigte Alkyl- oder Alkylenkette mit 10 bis 30 Kohlenstoffatomen aufweisen, und den Amiden ausgewählt ist,

wobei die Zusammensetzung nicht mehr als 4 Gew.-% grenzflächenaktive Stoffe, bezogen auf das Gesamtgewicht der Zusammensetzung, enthält.

2. Zusammensetzung nach Anspruch 1, wobei es sich bei den Copolymeren um Zweiblock-Copolymere, Dreiblock-Copolymere oder Multiblock-Copolymere handelt, die linear oder sternförmig verzweigt sind.

3. Zusammensetzung nach einem der vorhergehenden Ansprüche, **dadurch gekennzeichnet, dass** der hydrophile Block oder die hydrophilen Blöcke mindestens 60 Gew.-% des Sequenzcopolymers ausmachen.

4. Zusammensetzung nach einem der vorhergehenden Ansprüche, **dadurch gekennzeichnet, dass** das Sequenzcopolymer linear ist und unter den Zweiblock-Copolymeren, Dreiblock-Copolymeren und Multiblock-Copolymeren ausgewählt ist.

5. Zusammensetzung nach Anspruch 4, **dadurch gekennzeichnet, dass** das lineare Sequenzcopolymer unter den Zweiblock-Copolymeren und Dreiblock-Copolymeren ausgewählt ist, die einen zentralen hydrophilen Block und zwei hydrophobe seitliche Blöcke aufweisen.

6. Zusammensetzung nach einem der vorhergehenden Ansprüche, **dadurch gekennzeichnet, dass** die hydrophilen Blöcke bis zu 25 Mol-%, vorzugsweise bis zu 10 Mol-% und idealerweise bis zu 5 Mol-% eines oder mehrerer in Wasser unlöslicher Monomere nach Anspruch 1 enthalten.

7. Zusammensetzung nach einem der vorhergehenden Ansprüche, **dadurch gekennzeichnet, dass** der oder die hydrophoben Blöcke bis zu 25 Mol-%, vorzugsweise bis zu 10 Mol-% und idealerweise bis zu 5 Mol-% eines oder mehrerer wasserlöslicher oder in Wasser dispergierbarer Monomere nach Anspruch 1 enthalten.

8. Zusammensetzung nach einem der vorhergehenden Ansprüche, **dadurch gekennzeichnet, dass** das oder die Sequenzcopolymer(e) unter Poly(styrol-b-acrylsäure), Poly(methylacrylamid-b-acrylsäure), Poly(quaternisiertes trimethylammoniumethylacrylat-b-acrylamid)-Zweiblockcopolymer; Poly(styrol-b-acrylsäure-b-styrol)-Dreiblockcopolymer ausgewählt sind.

9. Zusammensetzung nach einem der vorhergehenden Ansprüche, **dadurch gekennzeichnet, dass** das oder die Sequenzcopolymer(e) in einem Mengenanteil von 0,01 bis 20 Gew.-% und vorzugsweise 0,1 bis 5 Gew.-%, bezogen auf das Gesamtgewicht der Zusammensetzung, enthalten sind.

10. Zusammensetzung nach einem der vorhergehenden Ansprüche, **dadurch gekennzeichnet, dass** das aminierte Silicon ausgewählt ist unter:

a) Polysiloxanen, die der folgenden Formel entsprechen:

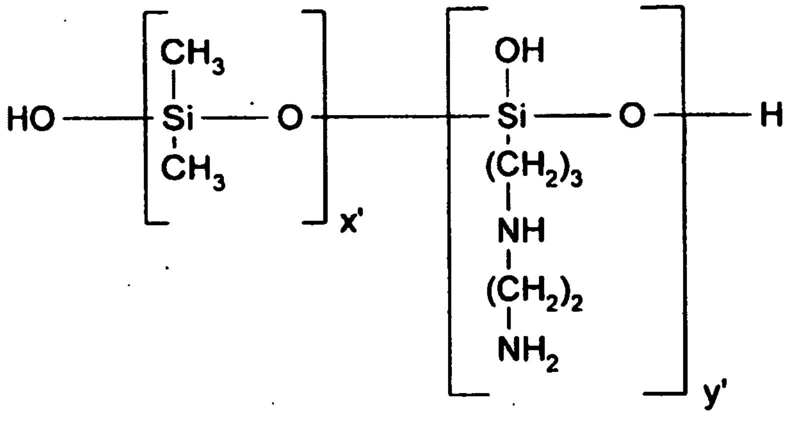

(I)

worin x' und y' ganze Zahlen sind, die so gewählt sind, dass die gewichtsmittlere Molmasse im Bereich von etwa 5.000 bis 500.000 liegt;
b) aminierten Siliconen der Formel:

$$R'_aG_{3-a}\text{-}Si(OSiG_2)_n\text{-}(OSiG_bR'_{2-b})_m\text{-}O\text{-}SiG_{3-a}\text{-}R'_a \qquad (II)$$

worin bedeuten:

die Gruppen G, die gleich oder verschieden sind, ein Wasserstoffatom, eine Phenylgruppe, OH, $C_{1-8}$-Alkyl oder $C_{1-8}$-Alkoxy,
die Indices a, die gleich oder verschieden sind, 0 oder eine ganze Zahl von 1 bis 3,
b 0 oder 1,
m und n Zahlen, die so sind, dass die Summe (n + m) insbesondere im Bereich von 1 bis 2 000 und besonders 50 bis 150 liegen kann, wobei n eine Zahl von 0 bis 1 999 und insbesondere 49 bis 149 bedeuten kann und m eine Zahl von 1 bis 2 000 und insbesondere 1 bis 10 sein kann;
die Gruppen R', die gleich oder verschieden sind, eine einwertige Gruppe der Formel $-CqH_2qL$, worin q eine ganze Zahl von 2 bis 8 ist und L eine gegebenenfalls quaternisierte, aminierte Gruppe ist, die unter den folgenden Gruppen ausgewählt ist:

$-NR''-Q-N(R'')_2$
$-N(R'')_2$
$-N^+(R'')_3$ A-
$-N^+H(R'')2$ A-
$-N^+H_2(R'')$ A-
$-N(R'')-Q-N^+R''H_2$ A-
$-NR''-Q-N^+ (R'')_2H$ A-
$-NR''-Q-N^+ (R'')_3$ A-,

worin R" Wasserstoff, Phenyl, Benzyl oder eine gesättigte einwertige Kohlenwasserstoffgruppe bedeuten kann; Q eine geradkettige oder verzweigte Gruppe der Formel $C_rH_{2r}$ ist, wobei r eine ganze Zahl von 2 bis 6 und vorzugsweise 2 bis 4 bedeutet; und A- ein Halogenid bedeutet,

c) aminierten Siliconen der folgenden Formel:

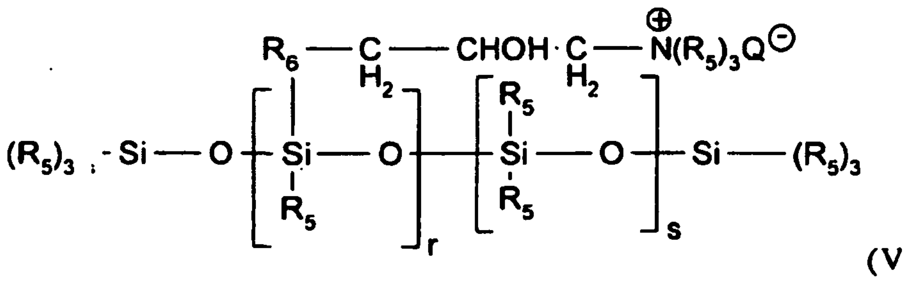

(VII)

in der Formel:

$R_5$ bedeutet eine einwertige Kohlenwasserstoffgruppe mit 1 bis 18 Kohlenstoffatomen;
$R_6$ bedeutet eine zweiwertige Kohlenwasserstoffgruppe, die über eine SiC-Bindung an Si gebunden ist;
Q- ist ein Anion, beispielsweise ein Halogenid oder das Salz einer organischen Säure;
r bedeutet einen statistischen Mittelwert von 2 bis 20;
s bedeutet einen statistischen Mittelwert von 20 bis 200;

d) quartären Ammoniumsiliconen der Formel (VIII);

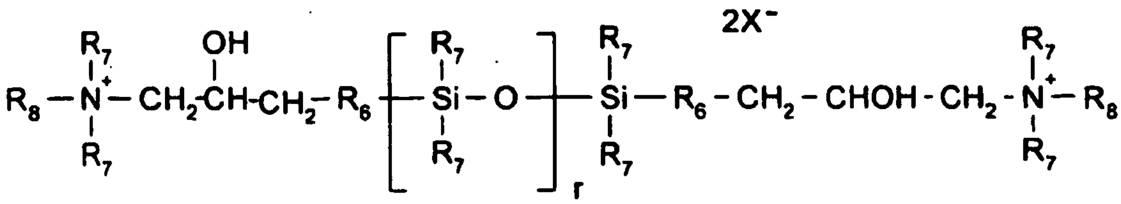

(VIII)

in der Formel:

die Gruppen $R_7$, die gleich oder verschieden sind, bedeuten eine einwertige Kohlenwasserstoffgruppe mit 1 bis 18 Kohlenstoffatomen;
$R_6$ bedeutet eine zweiwertige Kohlenwasserstoffgruppe, die über eine SiC-Bindung an Si gebunden ist;
die Gruppen $R_8$, die gleich oder verschieden sind, bedeuten ein Wasserstoffatom, eine einwertige Kohlenwasserstoffgruppe mit 1 bis 18 Kohlenstoffatomen;
X- ist ein Anion, beispielsweise ein Halogenid oder ein Salz einer organischen Säure;
r bedeutet einen statistischen Mittelwert von 2 bis 200;

e) aminierten Siliconen der Formel (IX):

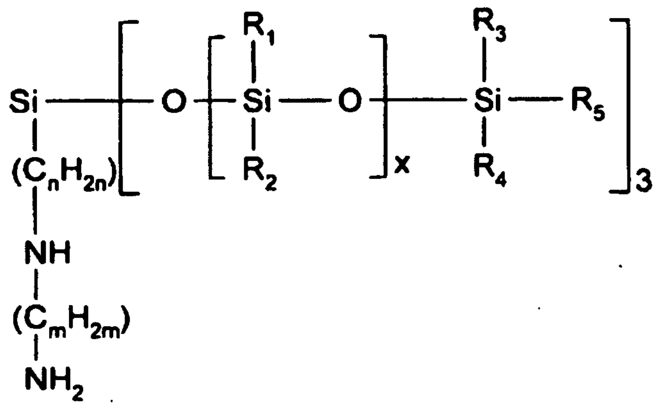

(IX)

in der Formel:

- $R_1$, $R_2$, $R_3$ und $R_4$, die gleich oder verschieden sind, bedeuten eine $C_{1-4}$-Alkylgruppe oder eine Phenylgruppe,
- $R_5$ bedeutet eine $C_{1-4}$-Alkylgruppe oder eine Hydroxygruppe,
- n ist eine ganze Zahl von 1 bis 5,
- m ist eine ganze Zahl von 1 bis 5,

wobei x so gewählt ist, dass der Amin-Index im Bereich von 0,01 bis 1 meq/g liegt;
f) polyalkoxylierten aminierten Siliconen vom Typ $(XY)_i$, wobei X ein Polysiloxanblock ist und Y ein Polyoxyalkylenblock ist, der mindestens eine Aminogruppe aufweist, die vorzugsweise aus Wiederholungseinheiten der folgenden allgemeinen Formel bestehen können:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \qquad (X)$$

in der Formel:

- a ist eine ganze Zahl größer oder gleich 1, vorzugsweise im Bereich von 5 bis 200 und besonders bevorzugt im Bereich von 5 bis 100,
- b ist eine ganze Zahl im Bereich von 0 bis 200, vorzugsweise im Bereich von 4 bis 200 und besonders bevorzugt im Bereich von 5 bis 100,
- x ist eine ganze Zahl im Bereich von 1 bis 10 000 und besonders bevorzugt 10 bis 5 000,
- die Gruppen R, die gleich oder verschieden sind, bedeuten eine zweiwertige organische Gruppe, die über eine Kohlenstoff-Silicium-Bindung an das angrenzende Siliciumatom und an ein Stickstoffatom gebunden ist,
- die Gruppen R', die gleich oder verschieden sind, bedeuten eine zweiwertige organische Gruppe, die über eine Kohlenstoff-Sauerstoff-Bindung an das angrenzende Sauerstoffatom und an ein Stickstoffatom gebunden ist.

**11.** Zusammensetzung nach einem der vorhergehenden Ansprüche, **dadurch gekennzeichnet, dass** das oder die aminierte(n) Silicon(e) in einem Mengenanteil von 0,01 bis 20 Gew.-% und vorzugsweise im Bereich von 0,1 bis 15 Gew.-% enthalten sind.

**12.** Zusammensetzung nach einem der vorhergehenden Ansprüche, **dadurch gekennzeichnet, dass** die Fettalkohole unter Stearylalkohol, Myristylalkohol, Cetylalkohol und deren Gemischen ausgewählt sind.

**13.** Zusammensetzung nach einem der vorhergehenden Ansprüche, **dadurch gekennzeichnet, dass** die Amide Amide mit $C_{12-22}$-Fettkette und vorzugsweise $C_{16-18}$-Fettkette sind.

**14.** Zusammensetzung nach einem der vorhergehenden Ansprüche, **dadurch gekennzeichnet, dass** das oder die

nicht polymeren Verdickungsmittel in einem Mengenanteil von 0,5 bis 10 Gew.-% und vorzugsweise 2 bis 6 Gew.-%, bezogen auf das Gesamtgewicht der Zusammensetzung, verwendet werden.

**15.** Zusammensetzung nach einem der vorhergehenden Ansprüche, **dadurch gekennzeichnet, dass** sie ferner kosmetische Additive und/oder Hilfsstoffe für die Formulierung enthält, wie anionische, kationische, amphotere oder nichtionische grenzflächenaktive Stoffe, ergänzende anionische, kationische, amphotere oder nichtionische Polymere, Perlglanzstoffe, Trübungsmittel, Pigmente und Farbstoffe, Parfums, pflanzlichen Öle, tierische Öle oder synthetische Öle, insbesondere ergänzende Silicone und Organosilicone, Silicongummis oder Siliconharze, Wachse, darunter Ceramide, organische oder anorganische Mikro- oder Nanopartikel, organische oder anorganische UV-Filter, Radikalfänger für freie Radikale, Vitamine, Peptide, Proteine, Aminosäuren, insbesondere basische Aminosäuren, Wirkstoffe gegen Haarausfall, antimykotische Wirkstoffe, Wirkstoffe für die Aktivierung des Haarwuchses, Weichmacher, Stoffe zur Einstellung und Fixierung des pH-Wertes, Antioxidantien, Salze, Lösemittel, Konservierungsmittel, Farbstoffvorprodukte von Haarfärbemitteln und Oxidationsmittel.

**16.** Zusammensetzung nach einem der vorhergehenden Ansprüche, **dadurch gekennzeichnet, dass** sie als dickflüssige Lotion, Gel, Creme oder Paste vorliegt.

**17.** Verwendung der Zusammensetzung nach einem der vorhergehenden Ansprüche für die Kämmbarkeit, die Glätte und den Glanz von Keratinfasern wie Haaren.

**18.** Verwendung der Zusammensetzung nach einem der vorhergehenden Ansprüche, um der Frisur gute kosmetische Eigenschaften zu geben, die nach mehreren Anwendungen bestehen bleiben.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0455185 A
- EP 1426038 A
- EP 1138317 A
- WO 2005039517 A
- FR 2548019
- EP 1312335 A
- EP 1120102 A
- EP 95238 A
- US 4185087 A
- EP 0530974 A


### Littérature non-brevet citée dans la description


- New Method of Polymer Synthesis. *Blackie Academic & Professional,* 1995, vol. 2, 1
- **C. J. Hawker.** *Trends Polym. Sci.,* 1996, vol. 4, 183
- **Matyjasezwski.** 117. *JACS,* 1995, 5614
- **Charmot D. ; Corpart P ; Adam H. ; Zard S.Z. ; Biadatti T. ; Bouhadir G.** *Macromol. Symp,* 2000, vol. 150, 23
- **D.A. Tomalia ; A.M. Naylor ; W.A. Goddard III.** *Angewandte Chemie, Int. Ed. Eng1.29,* 1990, 138-175
- **C.J. Hawker ; J.M.J. Frechet.** *J.Am. Chem Soc.,* 1990, vol. 112, 7638
- **B I Volt.** *Acta Polymer.,* 1995, vol. 46, 87-99
- **N.Ardoin ; D. Astruc.** *Bull. Soc. Chim. Fr,* 1995, vol. 132, 875-909
- **G.R. Newkome ; C.N. Moorefield ; F. VÖgtle.** *Dendritic Molecules,* 1996